(19) Europäisches Patentamt
European Patent Office
Office européen des brevets

(11) EP 4 781 993 A1

(12) **EUROPEAN PATENT APPLICATION**
published in accordance with Art. 153(4) EPC

(43) Date of publication:
**29.07.2026 Bulletin 2026/31**

(21) Application number: **24867118.2**

(22) Date of filing: **01.08.2024**

(51) International Patent Classification (IPC):
***A61K 31/545*** *(2006.01)* ***A61K 31/454*** *(2006.01)*
***A61K 45/06*** *(2006.01)* ***A61P 31/04*** *(2006.01)*

(52) Cooperative Patent Classification (CPC):
Y02A 50/30

(86) International application number:
**PCT/CN2024/109115**

(87) International publication number:
**WO 2025/060701 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.09.2023 CN 202311205221**

(71) Applicant: **Shenzhen China Resources Gosun Pharmaceuticals Co., Ltd.**
**Shenzhen, Guangdong 518049 (CN)**

(72) Inventors:
- **HU, Fupin**
  **Shanghai 200040 (CN)**
- **HUANG, Quanhua**
  **Shenzhen, Guangdong 518049 (CN)**
- **GUO, Yan**
  **Shanghai 200040 (CN)**
- **ZHANG, Yong**
  **Shenzhen, Guangdong 518049 (CN)**
- **WU, Shi**
  **Shanghai 200040 (CN)**
- **ZHANG, Weiwei**
  **Shenzhen, Guangdong 518049 (CN)**
- **HAN, Renru**
  **Shanghai 200040 (CN)**
- **LI, Xin**
  **Shanghai 200040 (CN)**
- **LAI, Baolin**
  **Shenzhen, Guangdong 518049 (CN)**
- **ZHONG, Yihua**
  **Shenzhen, Guangdong 518049 (CN)**

(74) Representative: **Dai, Simin**
**Reyda IP**
**A073**
**157, Quai du Président Roosevelt**
**92130 Issy-les-Moulineaux (FR)**

# (54) ANTIBACTERIAL COMPOSITION AND ANTIBACTERIAL DRUG

(57) An antibacterial composition and an antibacterial drug. The antibacterial composition comprises cephalosporin and zidebactam, wherein cephalosporin comprises at least one of ceftobiprole and a ceftobiprole derivative; and cephalosporin is calculated on the basis of $C_{20}H_{22}N_8O_6S_2$, and the mass ratio of the cephalosporin to zidebactam is 0.1:1 to 10:1. The compounding of cephalosporin and zidebactam enables cephalosporin and zidebactam to exhibit a synergistic effect, thereby providing the antibacterial composition with an excellent antibacterial activity, and a good antibacterial activity on bacteria resistant to the single drug of cephalosporin or zidebactam.

EP 4 781 993 A1

## Description

The present application claims priority to the Chinese patent application 202311205221.8 filed on September 18, 2023, and the contents of the Chinese patent application are incorporated herein by reference in their entirety.

## TECHNICAL FIELD

The present disclosure belongs to the technical field of antibacterial drugs, and specifically relates to an antibacterial composition and an antibacterial drug.

## BACKGROUND

Ceftobiprole is a broad-spectrum cephalosporin that exerts bactericidal activity in susceptible strains by binding to penicillin binding proteins (PBPs). The PBPs bound by ceftobiprole include PBPs with decreased susceptibility to multiple β-lactams, such as PBP2a in methicillin-resistant *Staphylococcus aureus* (MRSA), and PBP2b and PBP2x in *Streptococcus pneumoniae* (penicillin-intermediate and -resistant), thereby endowing ceftobiprole with activity against methicillin-resistant *Staphylococcus aureus* (MRSA) and against Gram-negative bacteria, including some *Pseudomonas aeruginosa.*

With the promotion and application of ceftobiprole in clinical practice, bacteria resistant to ceftobiprole have gradually emerged, such as Klebsiella pneumoniae carbapenemase (KPC) producing *Klebsiella pneumoniae,* resulting in reduced or no antibacterial activity of ceftobiprole against some bacteria.

## SUMMARY

The purpose of the present disclosure is to provide an antibacterial composition or an antibacterial kit, as well as an antibacterial drug, so as to further enhance the antibacterial effect against ceftobiprole-resistant bacteria, and to address the technical problem currently observed in which ceftobiprole exhibits reduced antibacterial activity or no antibacterial activity against some bacteria.

In the first aspect, an embodiment of the present disclosure provides an antibacterial composition or an antibacterial kit, wherein the antibacterial composition comprises a cephalosporin and zidebactam;

wherein the cephalosporin comprises one or more of ceftobiprole or a ceftobiprole derivative;

the cephalosporin is calculated as $C_{20}H_{22}N_8O_6S_2$, and the mass ratio of the cephalosporin to zidebactam is 0.1:1 to 10:1.

The antibacterial composition or the antibacterial kit of the embodiment of the present disclosure, through the compounding of the cephalosporin and zidebactam and simultaneous control of the mass ratio of the cephalosporin to zidebactam, enables the cephalosporin and zidebactam to exert a synergistic antibacterial effect on each other, thereby increasing the antibacterial activity of the antibacterial composition, making the antibacterial composition exhibit excellent antibacterial effects, especially the antibacterial effect against ceftobiprole-resistant bacteria.

In the second aspect, an embodiment of the present disclosure provides an antibacterial drug, wherein the antibacterial drug comprises the aforementioned antibacterial composition of the present disclosure.

Owing to the excellent antibacterial activity exhibited by the antibacterial composition of the present disclosure, the antibacterial drug of the present disclosure correspondingly possesses such excellent antibacterial activity as well.

## DETAILED DESCRIPTION OF THE PREFERRED EMBODIMENT

In order to make the technical problems to be solved, technical solutions, and beneficial effects of the present disclosure more clear and comprehensible. Hereinafter, the present disclosure will be further elaborated on in conjunction with the embodiments. It should be understood that the specific embodiments described herein are solely for illustrating the present disclosure and are not intended to limit the scope of the present disclosure.

In the present disclosure, "at least one" means one or more, and "more than one" means two or more. "At least one of the following (item(s))" or similar expressions refers to any combination of these items, including any combination of single item(s) or multiple item(s). For example, "at least one (item) of a, b, or c", or "at least one (item) of a, b, and c", may each indicate: a, b, c, a-b *(i.e.,* a and b), a-c, b-c, or a-b-c, wherein a, b, and c may each be single or may be multiple.

It should be understood that in various embodiments of the present disclosure, the magnitude of the serial numbers of the aforementioned processes does not indicate the order of execution; some or all of the steps may be executed in parallel or in sequence; the execution order of each process should be determined by its function and intrinsic logic, and should not constitute any limitation on the implementation process of the embodiments of the present disclosure.

The terms used in the embodiments of the present disclosure are solely for the purpose of describing specific

embodiments and are not intended to limit the present disclosure. The singular forms "a", "the", and "said" used in the embodiments of the present disclosure and the appended claims are also intended to include the plural form, unless the context clearly indicates otherwise.

The weights of relevant components mentioned in the embodiments of the specification of the present disclosure may refer not merely to the specific content of each component, but may also indicate the proportional relationship of weights among each component; therefore, any proportional scaling up or scaling down of the component contents specified in the embodiments of the specification of the present disclosure is deemed to be within the scope disclosed in the embodiments of the specification of the present disclosure. Specifically, the mass described in the embodiments of the specification of the present disclosure may be represented by mass units commonly known in the chemical engineering field, such as μg, mg, g, or kg.

Ceftobiprole is a new-generation broad-spectrum cephalosporin that exhibitsantibacterial activity against methicillin-resistant Staphylococcus *aureus* and against Gram-negative bacteria (including some *Pseudomonas aeruginosa)*. Its mechanism of action is similar to other cephalosporins, as a bactericide during the reproductive phase, it binds to PBPs, interferes with bacterial cell wall synthesis, inhibits bacterial growth, and ultimately causes bacterial death. However, with the clinical application of ceftobiprole, bacterial resistance to ceftobiprole has been increasingly enhanced. To resolve the technical problem in the prior art that ceftobiprole shows reduced antibacterial activity or no antibacterial activity against drug-resistant bacteria, the embodiments of the present disclosure propose the following technical solutions.

In the first aspect, an embodiment of the present disclosure provides an antibacterial composition or an antibacterial kit. The antibacterial composition or the antibacterial kit comprises a cephalosporin and zidebactam.

Among them, the cephalosporin comprises at least one of ceftobiprole or a ceftobiprole derivative, and may comprise either a single active ingredient, ceftobiprole or a ceftobiprole derivative, or may comprise both active ingredients simultaneously, namely ceftobiprole and a ceftobiprole derivative.

The cephalosporin is calculated as $C_{20}H_{22}N_8O_6S_2$, and the mass ratio of the cephalosporin to zidebactam is 0.1:1 to 10:1.

When the cephalosporin comprises ceftobiprole, the molecular formula of ceftobiprole is $C_{20}H_{22}N_8O_6S_2$, and the chemical name is (6R,7R)-7-((Z)-2-(5-amino-1,2,4-thiadiazol-3-yl)-2-(hydroxyimino)acetamido)-8-oxo-3-((*E*)-((*R*)-2-oxo-[1,3'-bipyrrolidin]-3-ylidene)methyl)-5-thia-1-azabicyclo[4.2.0]oct-2-ene-2-carboxylic acid, and the structural formula is shown in formula (1):

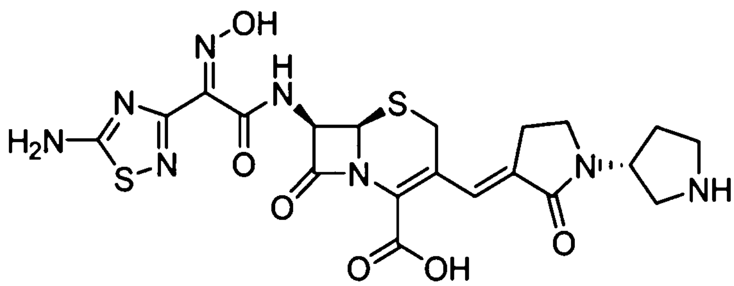

Formula (1)

When the cephalosporin comprises a ceftobiprole derivative, the ceftobiprole derivative refers to a derivative based on the aforementioned ceftobiprole.

The chemical name of zidebactam, as comprised in the antibacterial composition or the antibacterial kit of the present disclosure, is (2S,5R)-7-oxo-2-(2-((R)-piperidine-3-carbonyl)hydrazine-1-carbonyl)-1,6-diazabicyclo[3.2.1]octan-6-yl hydrogen sulfate. The structural formula of zidebactam is shown in formula (2):

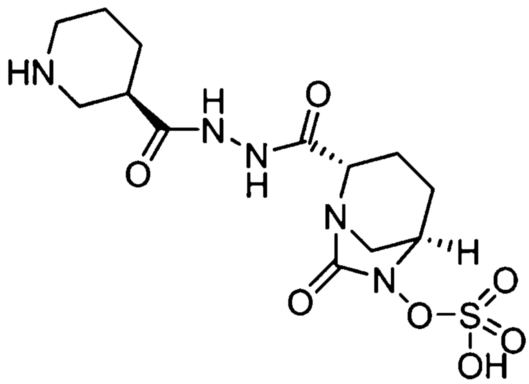

Formula (2)

Furthermore, zidebactam may also be a derivative of the structure shown in the aforementioned formula (2), exhibiting activity similar to that of the aforementioned formula (2).

The antibacterial composition or the antibacterial kit of the embodiments of the present disclosure, through the compounding of the cephalosporin and zidebactam and simultaneous control of the mass ratio of the cephalosporin to zidebactam within a specific range, enables the cephalosporin and zidebactam to exert a synergistic effect on each other, thereby enhancing the antibacterial effect of the antibacterial composition or the antibacterial kit, making the antibacterial composition or the antibacterial kit exhibit excellent antibacterial activity against bacteria resistant to cephalosporin monotherapy or to zidebactam monotherapy.

In some embodiments, in the antibacterial composition or the antibacterial kit, the cephalosporin derivative is calculated as ceftobiprole (molecular formula $C_{20}H_{22}N_8O_6S_2$), and the mass ratio of the cephalosporin to zidebactam may further be 0.1:1 to 8:1; in further exemplary embodiments, it may be 0.1:1 to 4:1; in still further exemplary embodiments, it may be 0.1:1 to 2.5:1. In specific exemplary embodiments, the mass ratio of the cephalosporin to zidebactam may be 0.1:1, 0.2:1, 0.3:1, 0.4:1, 0.5:1, 0.6:1, 0.7:1, 0.8:1, 0.9:1, 1:1, 1.5:1, 1.6:1, 1.7:1, 1.8:1, 1.9:1, 2:1, 2.1:1, 2.2:1, 2.3:1, 2.4:1, 2.5:1, 3:1, 4:1, 5:1, 6:1, 7:1, 8:1, 9:1, 10:1 and other typical but non-limiting mass ratios. Among them, when the cephalosporin comprises solely ceftobiprole, the mass ratio refers to the mass ratio of ceftobiprole calculated as $C_{20}H_{22}N_8O_6S_2$ to zidebactam; when the cephalosporin comprises solely a ceftobiprole derivative, the mass ratio refers to the mass ratio of the ceftobiprole derivative calculated as $C_{20}H_{22}N_8O_6S_2$ to zidebactam. When the cephalosporin comprises ceftobiprole and the ceftobiprole derivative, the mass ratio refers to the mass ratio of the total amount of ceftobiprole and the ceftobiprole derivative, calculated as $C_{20}H_{22}N_8O_6S_2$, to zidebactam.

Controlling the mass ratio of the cephalosporin to zidebactam within this range further promotes the synergistic effect on each other between the cephalosporin and zidebactam, thereby further enhancing the antibacterial activity of the antibacterial composition or the antibacterial kit.

In some embodiments, the cephalosporin and zidebactam comprised in the antibacterial composition or the antibacterial kit may be provided in a mixed form. By providing the cephalosporin and zidebactam in a mixed form, it is conducive to enhancing the product uniformity of the antibacterial composition or the antibacterial kit, while facilitating the use of the antibacterial composition or the antibacterial kit, such that the antibacterial composition or the antibacterial kit does not need to be mixed again prior to use.

In some embodiments, the cephalosporin and zidebactam exert a synergistic effect against bacteria.

In some embodiments, the cephalosporin and zidebactam exert a fractional inhibitory concentration index (FIC) of less than or equal to 0.5 against the bacteria, preferably less than or equal to 0.2, more preferably less than or equal to 0.15, or less than or equal to 0.13, for example, an FIC of 0.13, 0.1, 0.09, 0.08, 0.07, 0.06, 0.05, 0.04, 0.03, 0.02, or 0.01.

In some embodiments, when the cephalosporin and zidebactam are used in combination, ceftobiprole has an $MIC_{50}$ of less than or equal to 4 mg/L against the bacteria, preferably an $MIC_{50}$ of less than or equal to 2 mg/L, less than or equal to 0.5 mg/L, less than or equal to 0.125 mg/L, or less than or equal to 0.06 mg/L.

In some embodiments, the bacteria are KPC-2 producing bacteria.

In some embodiments, when the cephalosporin and zidebactam are used in combination, ceftobiprole has an $MIC_{90}$ of less than or equal to 8 mg/L against the bacteria, preferably an $MIC_{90}$ of less than or equal to 4 mg/L, less than or equal to 2 mg/L, less than or equal to 1 mg/L, less than or equal to 0.5 mg/L, or less than or equal to 0.25 mg/L.

In some embodiments, the bacteria are OXA-48 producing bacteria.

In some embodiments, the bacteria are Gram-negative bacteria.

In some embodiments, the bacteria are *Escherichia coli* and/or *Klebsiella pneumoniae;*
in some embodiments, the bacteria are one or more of NDM producing bacteria, KPC producing bacteria, or OXA producing bacteria.

In some embodiments, the cephalosporin and zidebactam in the antibacterial composition or the antibacterial kit may also be provided separately from each other and then mixed prior to use. For example, when the antibacterial composition is prepared as an injection, the cephalosporin and zidebactam can be packaged separately and then mixed together prior to injection, or the cephalosporin and zidebactam can be injected separately. By means of providing the cephalosporin and zidebactam separately, the qualitystability of each component in the antibacterial composition is further enhanced.

In some embodiments, when the cephalosporin comprises a ceftobiprole derivative, the ceftobiprole derivative may be a compound capable of being converted into ceftobiprole. In specific exemplary embodiments, the ceftobiprole derivative may comprise at least one of a ceftobiprole salt, a ceftobiprole ester, or a ceftobiprole ester derivative.

Among them, the ceftobiprole salt is a salt formed based on ceftobiprole, such as an alkali metal salt, hydrochloride, bromate, acetate, sulfate, and the like of ceftobiprole. In further exemplary embodiments, the alkali metal salt of ceftobiprole may be a sodium salt or a potassium salt. The ceftobiprole ester is a compound containing an ester group formed based on ceftobiprole.

In some embodiments, the ceftobiprole ester may be a compound having the structure as shown in formula (3), the molecular formula of which is $C_{26}H_{26}N_8O_{11}S_2$, and the chemical name is (6R,7R)-7-((Z)-2-(5-amino-1,2,4-thiadiazol-3-yl)-2-(hydroxyimino)acetamido]-3-((*E*)-((*R*)-1'-(((5-methyl-2-oxo-1,3-dioxol-4-yl)methoxy)carbonyl)-2-oxo-[1,3'-bipyrrolidin]-3-ylidene)methyl)-8-oxo-5-thia-1-azabicyclo[4.2.0]oct-2-ene-2-carboxylic acid.

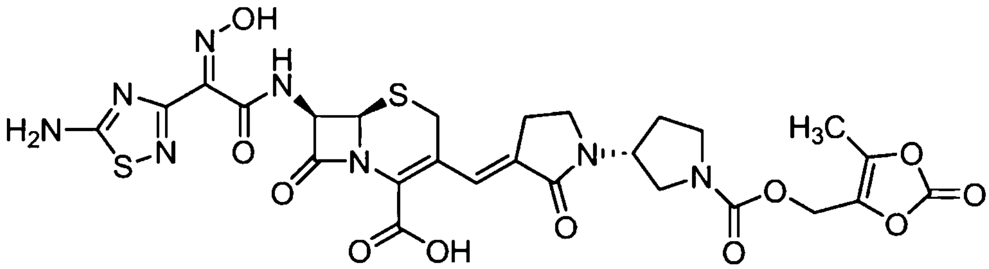

Formula (3)

The ceftobiprole ester shown in formula (3), as a prodrug of ceftobiprole, hydrolyzes into ceftobiprole after entering the human body; the ceftobiprole ester shown in formula (3) is conducive to enhancing the stability of the compound.

In some embodiments, the ceftobiprole ester derivative may also comprise a ceftobiprole ester salt formed from a ceftobiprole ester; in further aspects, the ceftobiprole ester salt may comprise at least one of an acetate, sulfate, hydrochloride, bromate, or alkali metal salt of a ceftobiprole ester. In a still further aspect, the alkali metal salt of the ceftobiprole ester may be a sodium salt or a potassium salt. The salt formed from ceftobiprole and a ceftobiprole ester can enhance the water solubility of ceftobiprole and the ceftobiprole ester, thereby, when the antibacterial composition is made into an injection, the injection has a short reconstitution time; meanwhile, the formation of sodium salt is conducive to reducing the irritation associated with injection administration.

In some embodiments, the sodium salt formed from the ceftobiprole ester shown in formula (3) may be ceftobiprole ester sodium. The molecular formula of ceftobiprole ester sodium is $C_{26}H_{25}N_8NaO_{11}S_2$, and the chemical name is monosodium (6R,7R)-7-((*Z*)-2-(5-amino-1,2,4-thiadiazol-3-yl)-2-(hydroxyimino)acetamido]-3-((*E*)-((*R*)-1'-(((5-methyl-2-oxo-1,3-dioxol-4-yl)methoxy)carbonyl)-2-oxo-[1,3'-bipyrrolidin]-3-ylidene)methyl)-8-oxo-5-thia-1-azabicyclo [4.2.0]oct-2-ene-2-carboxylate. The structural formula of ceftobiprole ester sodium is as shown in formula (4):

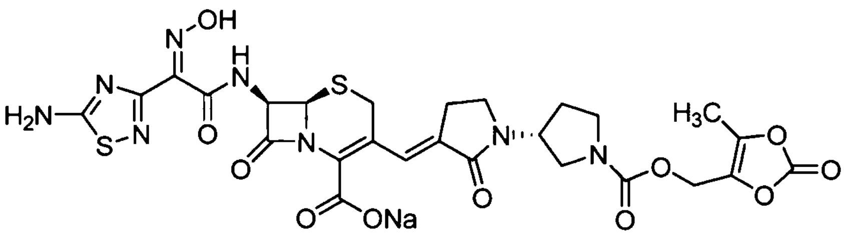

(4)

After the ceftobiprole ester shown in formula (3) forms the ceftobiprole ester sodium shown in formula (4), the water solubility is enhanced, thereby further enhancing the water solubility of the antibacterial composition and simultaneously reducing the irritation when the antibacterial composition is used as an injection, such as for intravenous injection.

In the second aspect, an embodiment of the present disclosure provides an antibacterial drug. The antibacterial drug comprises the aforementioned antibacterial composition of the present disclosure. Since the antibacterial composition of the present disclosure exhibits excellent antibacterial activity, the antibacterial drug thus exhibits excellent antibacterial activity.

In some embodiments, the antibacterial drug further comprises a pharmaceutically acceptable excipient, such as a solvent, a bulking agent, or a pH regulator.

In some embodiments, the antibacterial drug may be an injection.

In some embodiments, the antibacterial drug comprises a drug used against at least one of NDM producing bacteria, KPC producing bacteria, or OXA producing bacteria.

In some embodiments, the NDM producing bacteria comprise *Escherichia coli.* In further embodiments, when the antibacterial drug is applied to NDM producing *Escherichia coli,* the mass ratio of the cephalosporin to zidebactam may be 0.1:1 to 10:1, preferably 0.1:1 to 8:1.

In some embodiments, the KPC producing bacteria comprise *Klebsiella pneumoniae.* In further embodiments, when the antibacterial drug is applied to KPC producing *Klebsiella pneumoniae,* the mass ratio of the cephalosporin to zidebactam may be 0.1:1 to 10:1, preferably 0.1:1 to 8:1.

In some embodiments, the OXA producing bacteria comprise *Klebsiella pneumoniae.* In further embodiments, when the antibacterial drug is applied to OXA producing *Klebsiella pneumoniae,* the mass ratio of the cephalosporin to zidebactam may be 0.1:1 to 10:1, preferably 0.1:1 to 8:1.

The antibacterial composition of the embodiments of the present disclosure exhibits excellent antibacterial activity against NDM producing *Escherichia coli,* KPC producing *Klebsiella pneumoniae,* OXA producing *Klebsiella pneumoniae,* enabling the antibacterial drug of the embodiments of the present disclosure to exert a favorable therapeutic effects on infections caused by such bacteria.

In some embodiments, the dosage of the antibacterial drug is: the concentration of zidebactam in the application environment is from 4 mg/L to 8 mg/L; in specific exemplary embodiments, the concentration of zidebactam may be 4 mg/L, 5 mg/L, 6 mg/L, 7 mg/L, or 8 mg/L. Controlling the concentration of zidebactam within this range further enhances the synergistic effect on each other between the cephalosporin and zidebactam. The term "concentration in the application environment" as referred to herein means the concentration level of the drug and its metabolite in a specific application environment, which can be a specific site in the human body, such as blood, tissue, or organ and the like, or it can be an *in vitro* environment, such as a drug formulation, a drug release system, or a drug delivery device. This concentration can be the concentration of the free (not bound to protein or other molecule) drug, or it can be the total drug concentration (including free and bound drugs). It is usually expressed as the mass of the drug and its metabolite in each liter of fluid (such as mg/L or μg/L).

In some embodiments, the antibacterial drug comprises a drug for treating at least one of pulmonary infection, bloodstream infection, or urinary tract infection. In further embodiments, treating pulmonary infection, bloodstream infection, and urinary tract infection can be caused by multidrug-resistant, extensively drug-resistant, or pan-drug-resistant bacteria.

In some embodiments, the aforementioned antibacterial composition or the antibacterial kit or the aforementioned antibacterial drug is used for treating pulmonary infection, bloodstream infection and/or urinary tract infection.

In a second aspect, an embodiment of the present disclosure provides a use of the aforementioned antibacterial drug in the manufacture of a medicament for treating pulmonary infection, bloodstream infection and/or urinary tract infection.

In a third aspect, an embodiment of the present disclosure provides a method of treating pulmonary infection, bloodstream infection and/or urinary tract infection, comprising administering the aforementioned antibacterial composition or kit or the aforementioned antibacterial drug to a subject in need thereof.

In a third aspect, an embodiment of the present disclosure provides a use of a therapeutically effective amount of a cephalosporin and a therapeutically effective amount of zidebactam in the manufacture of a medicament, drug combination, or kit for treating pulmonary infection, bloodstream infection and/or urinary tract infection; wherein the therapeutically effective amount of the cephalosporin and zidebactam may be administered simultaneously, separately, or sequentially;

wherein the cephalosporin comprises one or more of ceftobiprole or a ceftobiprole derivative;
the cephalosporin is calculated as $C_{20}H_{22}N_8O_6S_2$, and the mass ratio of the cephalosporin to zidebactam is 0.1:1 to 10:1, preferably 0.1:1 to 8:1.

The antibacterial compositions and uses thereof and antibacterial drugs and the like in the embodiments of the present disclosure are illustrated below through multiple specific examples.

## Example 1

As recommended by CLSI (Clinical and Laboratory Standards Institute) M07-A11 (2018 edition), the Broth-microdilution method is adopted to determine the minimum inhibitory concentration (MIC) of ceftobiprole and zidebactam against clinical isolates. It should be noted that, in the present disclosure, $MIC_{50}$ refers to the minimum drug concentration that inhibits the growth of 50% of the tested bacteria, and $MIC_{90}$ refers to the minimum drug concentration that inhibits the growth of 90% of the tested bacteria.

Antibacterial drug: ceftobiprole, manufacturer: Shenyang Sanjiu Pharmaceutical, content: 92.1%, batch number: YF/BALP141/000. Zidebactam, manufacturer: Target M01 company, batch number: 149661. Zidebactam, as described above, the English name is Zidebactam, and the chemical name is: (2S,5R)-7-oxo-2-(2-((R)-piperidine-3-carbonyl)hydrazine-1-carbonyl)-1,6-diazabicyclo[3.2.1]octan-6-yl hydrogen sulfate.

Tested strains: a total of 24 genotypic strains producing KPC or OXA β-lactamase. Specifically, the tested strains include 24 strains *of Klebsiella pneumoniae,* including 12 KPC-2 producing strains and 12 OXA-232 producing strains.

Quality control strains: *Escherichia coli* ATCC 25922, (KPC-2 producing) *Klebsiella pneumoniae* ATCC BAA-1705, and (NDM-1 producing) *Klebsiella pneumoniae* ATCC BAA-2146.

Medium: cation-adjusted Mueller-Hinton broth (CAMHB), a product of BBL Company, USA.

Inoculum size: Pure isolated colonies were adjusted with normal saline to 0.5 McFarland turbidity. After appropriate dilution, the suspension was added to the wells of a 96-well U-bottom microplate (containing 50 μL antibacterial drug solution). The inoculum size was $5 \times 10^5$ CFU/mL.

Culture conditions: air environment, 35°C ± 2°C, 16 to 20 hours.

Preparation of Antibacterial Drug Solution : Ceftobiprole and zidebactam were taken separately, dissolution and dilution were performed according to the antibacterial drug reference to the CLSI standards and the drug package insert, A1 to A6, a total of 6 groups of antibacterial drug solutions, were prepared, among which, group A1 was the control group containing solely the single agent ceftobiprole, and group A2 was the control group containing solely the single agent zidebactam. Different antibacterial drug solutions with ceftobiprole concentrations ranging from 128 mg/L to 0.06 mg/L were respectively included in groups A1 and A3 to A6, and different antibacterial drug solutions with zidebactam concentrations ranging from 128 mg/L to 0.06 mg/L were included in group A2. The specific concentrations within the range of 128 mg/L to 0.06 mg/L in groups A1 to A6 were respectively 128 mg/L, 64 mg/L, 32 mg/L, 16 mg/L, 8 mg/L, 4 mg/L, 2 mg/L, 1 mg/L, 0.5 mg/L, 0.25 mg/L, 0.125 mg/L, or 0.06 mg/L, respectively. The components contained in the antibacterial drug solutions of groups A1 to A6 are shown in Table 1.

Table 1. Concentrations of ceftobiprole/zidebactam contained in the antibacterial drug solutions of groups A1 to A6

| Group | Concentration of ceftobiprole | Concentration of zidebactam | Concentration ratio (ceftobiprole: zidebactam) |
|---|---|---|---|
| Group A1 | 128 mg/L to 0.06 mg/L | / | / |
| Group A2 | / | 128 mg/L to 0.06 mg/L | / |
| Group A3 | 128 mg/L to 0.06 mg/L | Adjusted based on concentration of ceftobiprole | 1:1 |
| Group A4 | 128 mg/L to 0.06 mg/L | Adjusted based on ceftobiprole concentration | 2:1 |
| Group A5 | 128 mg/L to 0.06 mg/L | Fixed at 4 mg/L | 128:4, 64:4, 32:4, 16:4, 8:4, 4:4, 2:4, 1:4, 0.5:4, 0.25:4, 0.125:4, 0.06:4 |
| Group A6 | 128 mg/L to 0.06 mg/L | Fixed at 8 mg/L | 128:8, 64:8, 32:8, 16:8, 8:8, 4:8, 2:4, 1:8, 0.5:8, 0.25:8, 0.125:8, 0.06:8 |
| Note: The concentrations in Table 1 are mass concentrations; therefore, the concentration ratio in Table 1 is equal to the mass ratio. | | | |

Experimental results: Currently, there are no established CLSI or EUCAST breakpoints for ceftobiprole/β-lactamase inhibitor. Therefore, the antibacterial activity of ceftobiprole/β-lactamase inhibitor is evaluated in accordance with the standards for ceftobiprole. WHONET 5.6 software is used to perform statistical analysis on the results of antibacterial susceptibility test. The fractional inhibitory concentration index (FIC) for the combination of ceftobiprole and zidebactam is calculated according to Equation (1). The results are shown in Tables 2 to 5.

$$\text{FIC index} = \frac{\text{MIC of drug A in combination}}{\text{MIC of drug A alone}} + \frac{\text{MIC of drug B in combination}}{\text{MIC of drug B alone}} \quad (1)$$

In Equation (1), drug A is ceftobiprole, and drug B is zidebactam. The synergistic effects of the combination are interpreted based on the following criteria:

(1) FIC ≤ 0.5: synergy, indicating that the antibacterial activity after the combination of the two drugs is significantly higher than that of each single agent;
(2) 0.5 < FIC ≤ 1: additive effect, indicating that the combination of the two drugs has slightly increased antibacterial activity compared to either single agent;
(3) 1 < FIC ≤ 2: indifferent effect, indicating that the activities of both antibacterial drugs are not affected by the other drug;
(4) FIC > 2: antagonism, indicating that the activity of one antibacterial drug is attenuated by the other antibacterial drug.

Table 2. *In vitro* antibacterial activity of ceftobiprole/zidebactam against the tested bacteria in Example 1

| Bacteria (strains) | Antibacterial drug | MIC (mg/L) | | | | FIC | Explanation |
|---|---|---|---|---|---|---|---|
| | | MIC range | MIC 50 | MIC 90 | Mode | | |
| KPC-2 producing *Klebsiella pneumoniae* (12 strains) | Group AI | >128 | >128 | >128 | >128 | Calculated as 128 | / |
| | Group A2 | 1 to >128 | >128 | >128 | >128 | Calculated as 128 | / |
| | Group A3 | 1 to 4 | 2 | 4 | 2 | 2/128+2/128=0. 03 | Synergy |
| | Group A4 | 2 to 8 | 2 | 8 | 2 | 2/128+1/128=0. 02 | Synergy |
| | Group A5 | ≤0.06 to 4 | 0.5 | 1 | 0.5 | 0.5/128+4/128= 0.03 | Synergy |
| | Group A6 | ≤0.06 to 2 | 0.12 5 | 1 | ≤0.06 | 0.125/128+8/12 8=0.07 | Synergy |
| OXA-48 producing *Klebsiella pneumoniae* (12 strains) | Group A1 | >128 | >128 | >128 | >128 | Calculated as 128 | / |
| | Group A2 | 1 to >128 | 64 | >128 | >128 | / | / |
| | Group A3 | 1 to 4 | 2 | 2 | 2 | 2/128+2/64=0.0 5 | Synergy |
| | Group A4 | 2 to 8 | 4 | 4 | 4 | 4/128+2/64=0.0 6 | Synergy |
| | Group A5 | ≤0.06 to 1 | ≤0.0 6 | 0.5 | ≤0.06 | 0.06/128+4/64= 0.06 | Synergy |
| | Group A6 | ≤0.06 to 0.25 | ≤0.0 6 | 0.25 | ≤0.06 | 0.06/128+8/64= 0.13 | Synergy |
| Note: In Table 2, the MIC for group A2 refers to the concentration of zidebactam, and the MIC for groups A1 and A3 to A6 refers to the concentration of ceftobiprole. | | | | | | | |

As shown in Table 2, in group A5, when the concentration of zidebactam is 4 mg/L, the $MIC_{50}$ of ceftobiprole against KPC-2 producing *Klebsiella pneumoniae* is 0.5 mg/L; at which point the concentration ratio of ceftobiprole to zidebactam is 0.5:4 *(i.e.,* 0.125:1); the $MIC_{50}$ of ceftobiprole against OXA-232 producing *Klebsiella pneumoniae* is less than or equal to 0.06 mg/L;and the concentration ratio of ceftobiprole to zidebactam is calculated as 0.06:4 *(i.e.,* 0.015:1).

In group A6, when the concentration of zidebactam is 8 mg/L, the $MIC_{50}$ of ceftobiprole against KPC-2 producing *Klebsiella pneumoniae* is 0.125 mg/L; at which point the concentration ratio of ceftobiprole to zidebactam is 0.125:4 *(i.e.,* 0.032:1); the $MIC_{50}$ of ceftobiprole against OXA-232 producing *Klebsiella pneumoniae* is less than or equal to 0.06 mg/L; the concentration ratio of ceftobiprole to zidebactam is calculated as 0.06:4 *(i.e.,* 0.015:1).

In addition, as shown in Table 2, the FICs values of groups A3 and A4 are all lower than that of group A6, indicating that under the condition that the mass ratio of ceftobiprole to zidebactam ranging from 1:1 to 2:1, the synergistic effect is superior to that of the 0.015:1 ratio in group A6. The antibacterial results of ceftobiprole/zidebactam for groups A1 to A6 are shown in Tables 3 to 5.

Table 3. Antibacterial results of ceftobiprole/zidebactam

| No. | Strain No. | Bacteria | Notes | Drug concentration | | | | | |
|---|---|---|---|---|---|---|---|---|---|
| | | | | Group A1 | Group A2 | Group A3 | Group A4 | Group A5 | Group A6 |
| 1 | 18-W09-043 | kpn | KPC-2 | >128 | >128 | 2 | 4 | 0.5 | 1 |
| 2 | 18-W09-045 | kpn | KPC-2 | >128 | >128 | 2 | 2 | 0.5 | 0.125 |
| 3 | 18-W09-046 | kpn | KPC-2 | >128 | 128 | 2 | 2 | 0.5 | ≤0.06 |
| 4 | 18-W09-047 | kpn | KPC-2 | >128 | 128 | 2 | 4 | ≤0.06 | ≤0.06 |
| 5 | 18-W09-049 | kpn | KPC-2 | >128 | >128 | 2 | 2 | 0.5 | ≤0.06 |
| 6 | 18-W09-050 | kpn | KPC-2 | >128 | 128 | 2 | 2 | 0.25 | 0.125 |
| 7 | 18-W09-051 | kpn | KPC-2 | >128 | >128 | 4 | 8 | 4 | 2 |
| 8 | 18-W13-043 | kpn | KPC-2 | >128 | >128 | 2 | 4 | ≤0.06 | ≤0.06 |
| 9 | 18-W13-044 | kpn | KPC-2 | >128 | >128 | 4 | 4 | 1 | 0.25 |
| 10 | 18-W13-045 | kpn | KPC-2 | >128 | 128 | 2 | 2 | 0.5 | 0.125 |
| 11 | 18-W13-046 | kpn | KPC-2 | >128 | >128 | 4 | 8 | 1 | 0.5 |
| 12 | 18-W13-047 | kpn | KPC-2 | >128 | 1 | 1 | 2 | ≤0.06 | ≤0.06 |
| 13 | 20-W4-001 | kpn | OXA-232 | >128 | >128 | 2 | 8 | 0.5 | ≤0.06 |
| 14 | 20-W4-002 | kpn | OXA-232 | >128 | >128 | 2 | 4 | 1 | ≤0.06 |
| 15 | 20-W4-003 | kpn | OXA-232 | >128 | 4 | 1 | 2 | ≤0.06 | ≤0.06 |
| 16 | 20-W4-004 | kpn | OXA-232 | >128 | >128 | 2 | 4 | ≤0.06 | 0.125 |
| 17 | 20-W4-005 | kpn | OXA-232 | >128 | 128 | 2 | 2 | ≤0.06 | ≤0.06 |
| 18 | 20-W4-007 | kpn | OXA-232 | >128 | 2 | 2 | 4 | ≤0.06 | ≤0.06 |
| 19 | 20-W4-008 | kpn | OXA-232 | >128 | 64 | 2 | 4 | ≤0.06 | 0.125 |
| 20 | 20-W4-009 | kpn | OXA-232 | >128 | 128 | 4 | 4 | ≤0.06 | 0.25 |
| 21 | 20-W4-010 | kpn | OXA-232 | >128 | 1 | 1 | 2 | ≤0.06 | 0.125 |
| 22 | 20-W4-011 | kpn | OXA-232 | >128 | 8 | 2 | 4 | ≤0.06 | ≤0.06 |
| 23 | 20-W4-012 | kpn | OXA-232 | >128 | >128 | 2 | 2 | 0.5 | 0.25 |
| 24 | 20-W4-013 | kpn | OXA-232 | >128 | 4 | 2 | 4 | ≤0.06 | ≤0.06 |
| Note: In Table 3, the drug concentration in group A2 refers to the concentration of zidebactam, and the drug concentrations in groups A1 and A3 to A6 are the concentrations of ceftobiprole. | | | | | | | | | |

Table 4. MIC distribution and cumulative inhibition rate of ceftobiprole/zidebactam against KPC producing *Klebsiella pneumoniae* in Example 1

| Antibacterial drug | | MIC (mg/L) | | | | | | | | | |
|---|---|---|---|---|---|---|---|---|---|---|---|
| | | 0.06 | 0.125 | 0.25 | 0.5 | 1 | 2 | 4 | 8 | 128 | >128 |
| Group A1 | Number of strains | | | | | | | | | | 12 |
| | Cumulative number of strains | | | | | | | | | | 12 |
| | Cumulative inhibition rate (%) | | | | | | | | | | 100 |
| Group A2 | Number of strains | | | | | 1 | | | | 4 | 7 |
| | Cumulative number of strains | | | | | 1 | | | | 5 | 12 |
| | Cumulative inhibition rate (%) | | | | | 8.3 | | | | 41.7 | 100 |
| Group A3 | Number of strains | | | | | 1 | 8 | 3 | | | |
| | Cumulative number of strains | | | | | 1 | 9 | 12 | | | |
| | Cumulative inhibition rate (%) | | | | | 8.3 | 75 | 100 | | | |

(continued)

| Antibacterial drug | | MIC (mg/L) | | | | | | | | | |
|---|---|---|---|---|---|---|---|---|---|---|---|
| | | 0.06 | 0.125 | 0.25 | 0.5 | 1 | 2 | 4 | 8 | 128 | >128 |
| Group A4 | Number of strains | | | | | | 6 | 4 | 2 | | |
| | Cumulative number of strains | | | | | | 6 | 10 | 12 | | |
| | Cumulative inhibition rate (%) | | | | | | 50 | 83.3 | 100 | | |
| Group A5 | Number of strains | 3 | | 1 | 5 | 2 | | 1 | | | |
| | Cumulative number of strains | 3 | | 4 | 9 | 11 | | 12 | | | |
| | Cumulative inhibition rate (%) | 25 | | 33.3 | 75 | 91.7 | | 100 | | | |
| Group A6 | Number of strains | 5 | 3 | 1 | 1 | 1 | 1 | | | | |
| | Cumulative number of strains | 5 | 8 | 9 | 10 | 11 | 12 | | | | |
| | Cumulative inhibition rate (%) | 41.7 | 66.7 | 75 | 83.3 | 91.7 | 100 | | | | |

Note: In Table 4, the MIC for group A2 refers to the concentration of zidebactam, and the MIC for groups A1 and A3 to A6 refers to the concentration of ceftobiprole.

Table 5. MIC distribution and cumulative inhibition rate of ceftobiprole/zidebactam against OXA-232 producing *Klebsiella pneumoniae* in Example 1

| Antibacterial drug | | MIC (mg/L) | | | | | | | | | | |
|---|---|---|---|---|---|---|---|---|---|---|---|---|
| | | 0.06 | 0.125 | 0.25 | 0.5 | 1 | 2 | 4 | 8 | 64 | 128 | >128 |
| Group A1 | Number of strains | | | | | | | | | | | 12 |
| | Cumulative number of strains | | | | | | | | | | | 12 |
| | Cumulative inhibition rate (%) | | | | | | | | | | | 100 |
| Group A2 | Number of strains | | | | | 1 | 1 | 2 | 1 | 1 | 2 | 4 |
| | Cumulative number of strains | | | | | 1 | 2 | 4 | 5 | 6 | 8 | 12 |
| | Cumulative inhibition rate (%) | | | | | 8.3 | 16.7 | 33.3 | 41.7 | 50 | 66.7 | 100 |
| Group A3 | Number of strains | | | | | 2 | 9 | 1 | | | | |
| | Cumulative number of strains | | | | | 2 | 11 | 12 | | | | |
| | Cumulative inhibition rate (%) | | | | | 16.7 | 91.7 | 100 | | | | |
| Group A4 | Number of strains | | | | | | 4 | 7 | 1 | | | |
| | Cumulative number of strains | | | | | | 4 | 11 | 12 | | | |
| | Cumulative inhibition rate (%) | | | | | | 33.3 | 91.7 | 100 | | | |

(continued)

| Antibacterial drug | | MIC (mg/L) | | | | | | | | | | |
|---|---|---|---|---|---|---|---|---|---|---|---|---|
| | | **0.06** | **0.125** | **0.25** | **0.5** | **1** | **2** | **4** | **8** | **64** | **128** | **>128** |
| Group A5 | Number of strains | 9 | | | 2 | 1 | | | | | | |
| | Cumulative number of strains | 9 | | | 11 | 12 | | | | | | |
| | Cumulative inhibition rate (%) | 75 | | | 91.7 | 100 | | | | | | |
| Group A6 | Number of strains | 7 | 3 | 2 | | | | | | | | |
| | Cumulative number of strains | 7 | 10 | 12 | | | | | | | | |
| | Cumulative inhibition rate (%) | 58.3 | 83.3 | 100 | | | | | | | | |
| Note: In Table 5, the MIC for group A2 refers to the concentration of zidebactam, and the MIC for groups A1 and A3 to A6 refers to the concentration of ceftobiprole. | | | | | | | | | | | | |

As shown in Tables 2 to 5, the $MIC_{50}$ and $MIC_{90}$ of ceftobiprole alone in group A1 against KPC producing *Klebsiella pneumoniae* and OXA-232 producing *Klebsiella pneumoniae* are both greater than or equal to 128 mg/L. This indicates that ceftobiprole alone exhibits no antibacterial activity against either KPC producing *Klebsiella pneumoniae* or OXA-232 producing *Klebsiella pneumoniae*.

**In** group A2, the $MIC_{50}$ and $MIC_{90}$ of zidebactam alone against KPC producing *Klebsiella pneumoniae* are both greater than 128 mg/L, and the $MIC_{50}$ and $MIC_{90}$ against OXA-232 producing *Klebsiella pneumoniae* are 64 mg/L and greater than 128 mg/L, respectively. This indicates that zidebactam alone exhibits no antibacterial activity against KPC producing *Klebsiella pneumoniae,* and exhibits weak antibacterial activity against OXA-232 producing *Klebsiella pneumoniae.*

For KPC producing *Klebsiella pneumoniae,* the $MIC_{50}$ of groups A3 and A4 are both 2 mg/L, the $MIC_{90}$ are 4 mg/L and 8 mg/L, respectively, and the $MIC_{50}$ of groups A5 and A6 are 0.5 mg/L and 0.125 mg/L, respectively, and the $MIC_{90}$ are both 1 mg/L. The combination of ceftobiprole and zidebactam exhibits excellent antibacterial activity against KPC producing *Klebsiella pneumoniae.* Compared with ceftobiprole alone, ceftobiprole and zidebactam combinations with different ratios formulations show a reduction in MIC90 ranging from greater than 16-fold to greater than 128-fold against KPC producing *Klebsiella pneumoniae,* and the combination of ceftobiprole and zidebactam exhibits extremely strong synergistic antibacterial activity.

For OXA-232 producing *Klebsiella pneumoniae,* the $MIC_{50}$ and $MIC_{90}$ of group A3 are both 2 mg/L, the $MIC_{50}$ and $MIC_{90}$ of group A4 are both 4 mg/L, the $MIC_{50}$ and $MIC_{90}$ of group A5 are less than or equal to 0.06 mg/L and 0.5 mg/L, respectively, and the $MIC_{50}$ and $MIC_{90}$ of group A6 are less than or equal to 0.06 mg/L and 0.25 mg/L, respectively. The combination of ceftobiprole and zidebactam exhibits excellent antibacterial activity against OXA-232 producing *Klebsiella pneumoniae.* Compared with ceftobiprole alone, ceftobiprole and zidebactam combinations with different ratios formulations show a reduction in $MIC_{90}$ ranging from greater than 32-fold to greater than 512-fold against OXA-232 producing *Klebsiella pneumoniae,* and the combination of ceftobiprole and zidebactam exhibits extremely strong synergistic antibacterial activity.

**Examples 2 to 7**

Examples 2 to 7 adopt the same method as Example 1 to examine the antibacterial activity against 24 strains of *Klebsiella pneumoniae* (including 12 KPC-2 producing strains and 12 OXA-232 producing strains) when the mass ratios of ceftobiprole to zidebactam are 1.5:1, 2.5:1, 4:1, 8:1, 10:1, and 0.1:1, respectively. The concentration range of ceftobiprole in Examples 2 to 7 is shown in Table 6, and the results are shown in Table 7. In Examples 1 to 7, the concentration ratio of ceftobiprole to zidebactam is the same as the mass ratio.

**Comparative Example A1**

Comparative Example A1 adopts the same method as Example 1 to examine the antibacterial activity against 24 strains of *Klebsiella pneumoniae* (including 12 KPC-2 producing strains and 12 OXA-232 producing strains) when a different ceftobiprole and zidebactam mass ratio is 11:1, the concentration range of ceftobiprole in Comparative Example A1 is shown in Table 6, and the results are shown in Table 7.

Table 6. Concentrations of ceftobiprole/zidebactam in Examples 2 to 7 and Comparative Example A1

| Group | Concentration of ceftobiprole | Concentration of zidebactam | Concentration ratio (ceftobiprole: zidebactam) |
|---|---|---|---|
| Example 2 | 128 mg/L to 0.06 mg/L | Adjusted based on concentration of ceftobiprole | 1.5:1 |
| Example 3 | 128 mg/L to 0.06 mg/L | Adjusted based on concentration of ceftobiprole | 2.5:1 |
| Example 4 | 128 mg/L to 0.06 mg/L | Adjusted based on concentration of ceftobiprole | 4:1 |
| Example 5 | 128 mg/L to 0.06 mg/L | Adjusted based on concentration of ceftobiprole | 8:1 |
| Example 6 | 128 mg/L to 0.06 mg/L | Adjusted based on concentration of ceftobiprole | 10:1 |
| Example 7 | 128 mg/L to 0.06 mg/L | Adjusted based on concentration of ceftobiprole | 0.1:1 |
| Comparative Example A1 | 128 mg/L to 0.06 mg/L | Adjusted based on concentration of ceftobiprole | 11:1 |

Table 7. *In vitro* antibacterial activity of ceftobiprole/zidebactam in Examples 2 to 7 and Comparative Example A1 against the tested bacteria

| Bacteria (strains) Bacteria (strains) | Example/ Comparative Example | Mass ratio of ceftobiprole and zidebactam | $MIC_{50}$ (ceftobiprole) | $MIC_{50}$ corresponding concentration of zidebactam | FIC |
|---|---|---|---|---|---|
| KPC-2 producing *Klebsiella pneumoniae* (12 strains) | Example 2 | 1.5:1 | 2 | 1.33 | 2/128+1.33/128 =0.03 |
| | Example 3 | 2.5:1 | 2 | 0.8 | 2/128+0.8/128 =0.02 |
| | Example 4 | 4:1 | 2 | 0.5 | 2/128+0.5/128 =0.02 |
| | Example 5 | 8:1 | 4 | 0.5 | 4/128+0.5/128 =0.04 |
| | Example 6 | 10:1 | 4 | 0.4 | 4/128+0.4/128 =0.03 |
| | Example 7 | 0.1:1 | 0.5 | 5 | 0.5/128+5/128 =0.04 |
| | Comparative Example A1 | 11:1 | 8 | 0.73 | 8/128+0.73/128 =0.07 |

(continued)

| Bacteria (strains) Bacteria (strains) | Example/ Comparative Example | Mass ratio of ceftobiprole and zidebactam | $MIC_{50}$ (ceftobiprole) | $MIC_{50}$ corresponding concentration of zidebactam | FIC |
|---|---|---|---|---|---|
| OXA-48 producing *Klebsiella pneumoniae* (12 strains) | Example 2 | 1.5:1 | 4 | 2.7 | 4/128+2.7/128 =0.05 |
| | Example 3 | 2.5:1 | 4 | 1.6 | 4/128+1.6/128 =0.04 |
| | Example 4 | 4:1 | 8 | 2 | 8/128+2/128=0 .08 |
| | Example 5 | 8:1 | 8 | 1 | 8/128+1/128=0 .07 |
| | Example 6 | 10:1 | 8 | 0.8 | 8/128+0.8/128 =0.07 |
| | Example 7 | 0.1:1 | 0.25 | 2.5 | 0.25/128+2.5/128=0.02 |
| | Comparative Example A1 | 11:1 | 16 | 1.5 | 16/128+1.5/128 =0.14 |
| Note: In Table 7, the MIC in Examples 2 to 7 and in Comparative Example A1 refers to the concentration of ceftobiprole. | | | | | |

As shown in Table 7, in the antibacterial activity of ceftobiprole against OXA-232 producing *Klebsiella pneumoniae* and KPC-2 producing *Klebsiella pneumoniae,* the FIC index under the condition that the mass ratio of ceftobiprole to zidebactam is 0.1:1 to 10:1 is less than the FIC index at the mass ratio of ceftobiprole to zidebactam of 11:1. This indicates that within the range of 0.1:1 to 10:1 for the mass ratio of ceftobiprole to zidebactam, there is a better synergistic effect.

**Comparative Example B1**

An investigation of the combined-use ratio of ceftobiprole and vaborbactam was conducted. The investigation method is the same as in the examples, with the solelydifference being that zidebactam in the antibacterial drug solutions of groups A2 to A6 in Example 1 is replaced with vaborbactam, and the numbering of groups A2 to A6 is correspondingly modified to groups B2 to B6.

The manufacturer of vaborbactam is GLP Bio company, batch number: GC193691. The chemical name of vaborbactam is (3*R*,6*S*)-2-hydroxy-3-[[2-(2-thienyl)acetyl]amino]-1,2-oxaborinane-6-acetic acid; the structural formula is shown in formula (5):

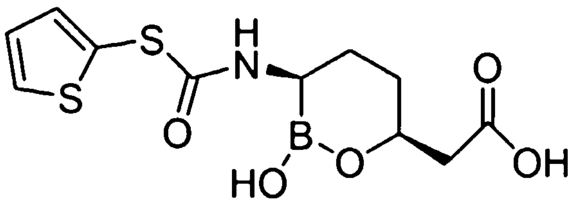

Formula (5)

The results of this comparative example are shown in Tables 8 to 9.

Table 8. *In vitro* antibacterial activity of ceftobiprole/vaborbactam against the tested bacteria

| Bacteria (strains) | Antibacterial drug | MIC (mg/L) | | | |
|---|---|---|---|---|---|
| | | MIC range | $MIC_{50}$ | $MIC_{90}$ | Mode |
| KPC-2 producing *Klebsiella pneumoniae* (12 strains) | Group B2 | >128 | >128 | >128 | >128 |
| | Group B3 | 32 to 64 | 32 | 64 | 32 |
| | Group B4 | 32 to 64 | 64 | 64 | 64 |
| | Group B5 | 64 to >128 | >128 | >128 | >128 |
| | Group B6 | 32 to >128 | >128 | >128 | >128 |
| OXA-48 producing *Klebsiella pneumoniae* (12 strains) | Group B2 | >128 | >128 | >128 | >128 |
| | Group B3 | 128 to >128 | 128 | >128 | 128, >128 |
| | Group B4 | 128 to >128 | >128 | >128 | >128 |
| | Group B5 | 128 to >128 | >128 | >128 | >128 |
| | Group B6 | 128 to >128 | >128 | >128 | >128 |
| Note: In Table 8, the MIC for group B2 refers to the concentration of vaborbactam, and the MIC for groups B3 to B6 refers to the concentration of ceftobiprole. | | | | | |

Table 9. MIC distribution and cumulative inhibition rate of ceftobiprole/vaborbactam against the tested bacteria

| Antibacterial drug | | KPC producing MIC (mg/L) | | | | OXA producing MIC (mg/L) | |
|---|---|---|---|---|---|---|---|
| | | 32 | 64 | 128 | >128 | 128 | >128 |
| Group B2 | Number of strains | | | | 12 | | 12 |
| | Cumulative number of strains | | | | 12 | | 12 |
| | Cumulative inhibition rate (%) | | | | 100 | | 100 |
| Group B3 | Number of strains | 8 | 4 | | | 6 | 6 |
| | Cumulative number of strains | 8 | 12 | | | 6 | 12 |
| | Cumulative inhibition rate (%) | 66.7 | 100 | | | 50 | 100 |
| Group B4 | Number of strains | 2 | 10 | | | 2 | 10 |
| | Cumulative number of strains | 2 | 12 | | | 2 | 12 |
| | Cumulative inhibition rate (%) | 16.7 | 100 | | | 16.7 | 100 |
| Group B5 | Number of strains | | 1 | 1 | 10 | 1 | 11 |
| | Cumulative number of strains | | 1 | 2 | 12 | 1 | 12 |
| | Cumulative inhibition rate (%) | | 8.3 | 16.7 | 100 | 8.3 | 100 |
| Group B6 | Number of strains | 1 | 1 | 1 | 9 | 1 | 11 |
| | Cumulative number of strains | 1 | 2 | 3 | 12 | 1 | 12 |
| | Cumulative inhibition rate (%) | 8.3 | 16.7 | 25 | 100 | 8.3 | 100 |
| Note: In Table 9, the MIC for group B2 refers to the concentration of vaborbactam, and the MIC for groups B3 to B6 refers to the concentration of ceftobiprole. | | | | | | | |

As shown in Tables 8 to 9, the $MIC_{50}$ and $MIC_{90}$ of vaborbactam alone in group B2 against KPC producing *Klebsiella pneumoniae* and OXA-232 producing *Klebsiella pneumoniae* are both greater than 128 mg/L, and vaborbactam alone exhibits no antibacterial activity against KPC producing *Klebsiella pneumoniae* and OXA-232 producing *Klebsiella pneumoniae.*

For KPC producing *Klebsiella pneumoniae,* the $MIC_{50}$ and $MIC_{90}$ of group B3 are 32 mg/L and 64 mg/L, respectively; the $MIC_{50}$ and $MIC_{90}$ of group B4 are both 64 mg/L; and the $MIC_{50}$ and $MIC_{90}$ of groups B5 and B6 are both greater than 128 mg/L. The combination of ceftobiprole and vaborbactam exhibits weak antibacterial activity against KPC producing *Klebsiella pneumoniae.*

For OXA-232 producing *Klebsiella pneumoniae,* the $MIC_{50}$ and $MIC_{90}$ of group B3 are 128 mg/L and greater than

128 mg/L, respectively, and the $MIC_{50}$ and $MIC_{90}$ of groups B3, B5, and B6 are all greater than 128 mg/L. The combination of ceftobiprole and vaborbactam exhibits no antibacterial activity against KPC producing *Klebsiella pneumoniae.*

**Comparative Example B2**

An investigation of the combined-use ratio of ceftobiprole and relebactam was conducted. The investigation method is the same as in the aforementioned examples, with the difference being that zidebactam in the antibacterial drug solutions of groups A2 to A6 in the aforementioned example is replaced with relebactam, and the numbering of groups A2 to A6 is correspondingly modified to groups C2 to C6.

The manufacturer of relebactam is Biochempartner, batch number: 20201209. The chemical name of relebactam is (1R,2S,5R)-7-oxo-2-(piperidin-4-ylcarbamoyl)-1,6-diazabicyclo[3.2.1]octan-6-yl hydrogen sulfate; the structural formula is shown in formula (6):

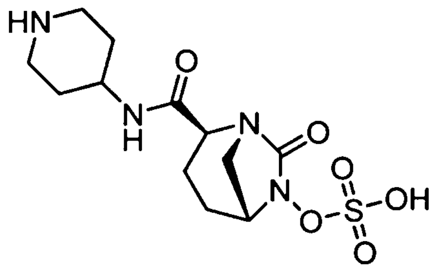

Formula (6)

The results of this comparative example are shown in Tables 10 to 11.

Table 10. *In vitro* antibacterial activity of ceftobiprole/relebactam against the tested bacteria

| **Bacteria (strains)** | **Antibacterial drug** | **MIC (mg/L)** | | | |
|---|---|---|---|---|---|
| | | **MIC range** | **$MIC_{50}$** | **$MIC_{90}$** | **Mode** |
| KPC-2 producing *Klebsiella pneumoniae (*12 strains) | Group C2 | 128 to >128 | >128 | >128 | >128 |
| | Group C3 | 8 to 32 | 16 | 32 | 16 |
| | Group C4 | 8 to 32 | 16 | 32 | 16 |
| | Group C5 | 8 to >128 | 32 | 128 | 32 |
| | Group C6 | 2 to 32 | 16 | 32 | 32 |
| OXA-48 producing | Group C2 | 128 to >128 | >128 | >128 | >128 |
| | Group C3 | 32 to 64 | 64 | 64 | 64 |
| *Klebsiella pneumoniae (*12 strains) | Group C4 | 32 to 64 | 64 | 64 | 64 |
| | Group C5 | 128 to >128 | >128 | >128 | >128 |
| | Group C6 | 128 to >128 | >128 | >128 | >128 |
| Note: In Table 10, the MIC for group C2 refers to the concentration of relebactam, and the MIC for groups C3 to C6 refers to the concentration of ceftobiprole. | | | | | |

Table 11. MIC distribution and cumulative inhibition rate of ceftobiprole/relebactam against the tested bacteria

| **Antibacterial drug** | **KPC producing** | | | | | | | | **OXA producing** | | | |
|---|---|---|---|---|---|---|---|---|---|---|---|---|
| | **MIC (mg/L)** | | | | | | | | **MIC (mg/L)** | | | |
| | **2** | **4** | **8** | **16** | **32** | **64** | **128** | **>128** | **32** | **64** | **128** | **>128** |
| Number of strains | | | | | | | 3 | 9 | | | 5 | 7 |

(continued)

| Antibacterial drug | | KPC producing | | | | | | | | OXA producing | | | |
|---|---|---|---|---|---|---|---|---|---|---|---|---|---|
| | | MIC (mg/L) | | | | | | | | MIC (mg/L) | | | |
| | | **2** | **4** | **8** | **16** | **32** | **64** | **128** | **>128** | **32** | **64** | **128** | **>128** |
| Group C2 | Cumulative number of strains | | | | | | | 3 | 12 | | | 5 | 12 |
| | Cumulative inhibition rate (%) | | | | | | | 25 | 100 | | | 41.7 | 100 |
| Group C3 | Number of strains | | | 3 | 7 | 2 | | | | 5 | 7 | | |
| | Cumulative number of strains | | | 3 | 10 | 12 | | | | 5 | 12 | | |
| | Cumulative inhibition rate (%) | | | 25 | 83.3 | 100 | | | | 41.7 | 100 | | |
| Group C4 | Number of strains | | | 2 | 7 | 3 | | | | 1 | 11 | | |
| | Cumulative number of strains | | | 2 | 9 | 12 | | | | 1 | 12 | | |
| | Cumulative inhibition rate (%) | | | 16.7 | 75 | 100 | | | | 8.3 | 100 | | |
| Group C5 | Number of strains | | | 2 | | 6 | 2 | 1 | 1 | | | 1 | 11 |
| | Cumulative number of strains | | | 2 | | 8 | 10 | 11 | 12 | | | 1 | 12 |
| | Cumulative inhibition rate (%) | | | 16.7 | | 66.7 | 83.3 | 91.7 | 100 | | | 8.3 | 100 |
| Group C6 | Number of strains | 1 | 1 | 1 | 4 | 5 | | | | | | 1 | 11 |
| | Cumulative number of strains | 1 | 2 | 3 | 7 | 12 | | | | | | 1 | 12 |
| | Cumulative inhibition rate (%) | 8.3 | 16.7 | 25 | 58.3 | 100 | | | | | | 8.3 | 100 |

Note: In Table 11, the MIC for group C2 refers to the concentration of relebactam, and the MIC for groups C3 to C6 refers to the concentration of ceftobiprole.

As shown in Tables 10 to 11, the $MIC_{50}$ and $MIC_{90}$ of relebactam alone in group C2 against KPC producing *Klebsiella pneumoniae* and OXA-232 producing *Klebsiella pneumoniae* are both greater than 128 mg/L, and relebactam alone exhibits no antibacterial activity against KPC producing *Klebsiella pneumoniae* and OXA-232 producing *Klebsiella pneumoniae.*

For KPC producing *Klebsiella pneumoniae,* the $MIC_{50}$ of groups C3, C4, and C6 are all 16 mg/L, the $MIC_{90}$ are all 16 mg/L, when ceftobiprole and relabactam are used in combination, the ratios of ceftobiprole to relabactam are 1:1 and 2:1, as well as when the concentration of relabactam is 8 mg/L, the antibacterial activity against KPC producing *Klebsiella pneumoniae* is general. The $MIC_{50}$ and $MIC_{90}$ of group C5 are 32 mg/L and 128 mg/L, respectively, when the concentration

of relebactam is 4 mg/L, the combination use of ceftobiprole and relebactam exhibits weak antibacterial activity against KPC producing *Klebsiella pneumoniae.*

For OXA-232 producing *Klebsiella pneumoniae,* the $MIC_{50}$ and $MIC_{90}$ of groups C3 and C4 are both 64 mg/L; when ceftobiprole and relebactam are used in combination, at ceftobiprole-to-relebactam ratios of 1:1 and 2:1, the antibacterial activity is weak. In groups C5 and C6, $MIC_{50}$ and $MIC_{90}$ are both greater than 128 mg/L; when the concentration of relebactam is 4 mg/L and 8 mg/L, the combination of ceftobiprole and relebactam has no antibacterial activity.

**Comparative Example B3**

An investigation of the combined-use ratio of ceftobiprole and taniborbactam was conducted. The investigation method is the same as in Example 1, with the difference being that zidebactam in the antibacterial drug solutions of groups A2 to A6 in Example 1 is replaced with taniborbactam, and the numbering of groups A2 to A6 is correspondingly modified to groups D2 to D6.

The manufacturer of taniborbactam is MCE company, batch number: 80267. The chemical name of taniborbactam is (*R*)-3-(2-((1*r*,4*R*)-4-((2-aminoethyl)amino)cyclohexyl)acetamido)-2-hydroxy-3,4-dihydro-2*H*-benzo[e][1,2]oxaborinine-8-carboxylic acid, and the structural formula is shown in formula (7):

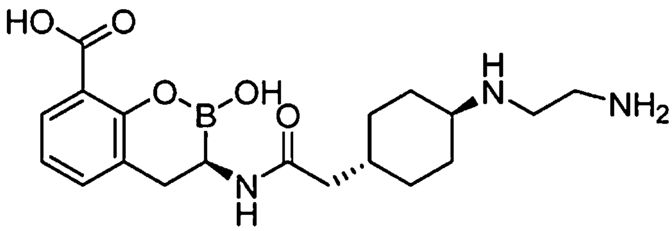

Formula (7)

The results of this comparative example are shown in Tables 12 to 13.

Table 12. *In vitro* antibacterial activity of ceftobiprole/taniborbactam against the tested bacteria

| Bacteria (strains) | Antibacterial drug | MIC (mg/L) | | | |
|---|---|---|---|---|---|
| | | MIC range | $MIC_{50}$ | $MIC_{90}$ | Mode |
| KPC-2 producing *Klebsiella pneumoniae* (12 strains) | Group D2 | >128 | >128 | >128 | >128 |
| | Group D3 | 4 to 32 | 8 | 16 | 8 |
| | Group D4 | 8 to 32 | 16 | 16 | 16 |
| | Group D5 | 4 to 32 | 16 | 32 | 16 |
| | Group D6 | 1 to 32 | 8 | 16 | 8 |
| OXA-48 producing *Klebsiella pneumoniae* (12 strains) | Group D2 | >128 | >128 | >128 | >128 |
| | Group D3 | 16 to 32 | 16 | 32 | 16 |
| | Group D4 | 16 to 32 | 32 | 32 | 32 |
| | Group D5 | 16 to 128 | 64 | 128 | 128 |
| | Group D6 | 8 to 64 | 32 | 64 | 64 |
| Note: In Table 12, the MIC for group D2 refers to the concentration of relebactam, and the MIC for groups D3 to D6 refers to the concentration of ceftobiprole. | | | | | |

Table 13. MIC distribution and cumulative inhibition rate of ceftobiprole/taniborbactam against KPC-2 producing and OXA-232 producing *Klebsiella pneumoniae*

| Antibacterial drug | | KPC producing | | | | | | OXA producing | | | | | |
|---|---|---|---|---|---|---|---|---|---|---|---|---|---|
| | | MIC (mg/L) | | | | | | MIC (mg/L) | | | | | |
| | | **1** | **4** | **8** | **16** | **32** | **>128** | **8** | **16** | **32** | **64** | **128** | **>128** |
| Group D2 | Number of strains | | | | | | 12 | | | | | | 12 |
| | Cumulative number of strains | | | | | | 12 | | | | | | 12 |
| | Cumulative inhibition rate (%) | | | | | | 100 | | | | | | 100 |
| Group D3 | Number of strains | | 2 | 8 | 1 | 1 | | | 8 | 4 | | | |
| | Cumulative number of strains | | 2 | 10 | 11 | 12 | | | 8 | 12 | | | |
| | Cumulative inhibition rate (%) | | 16.7 | 83.3 | 91.7 | 100 | | | 66.7 | 100 | | | |
| Group D4 | Number of strains | | | 4 | 7 | 1 | | | 1 | 11 | | | |
| | Cumulative number of strains | | | 4 | 11 | 12 | | | 1 | 12 | | | |
| | Cumulative inhibition rate (%) | | | 33.3 | 91.7 | 100 | | | 8.3 | 100 | | | |
| Group D5 | Number of strains | | 1 | 2 | 7 | 2 | | | 1 | | 5 | 6 | |
| | Cumulative number of strains | | 1 | 3 | 10 | 12 | | | 1 | | 6 | 12 | |
| | Cumulative inhibition rate (%) | | 8.3 | 25 | 83.3 | 100 | | | 8.3 | | 50 | 100 | |
| Group D6 | Number of strains | 2 | 2 | 6 | 1 | 1 | | 1 | | 5 | 6 | | |
| | Cumulative number of strains | 2 | 4 | 10 | 11 | 12 | | 1 | | 6 | 12 | | |
| | Cumulative inhibition rate (%) | 16.7 | 33.3 | 83.3 | 91.7 | 100 | 8.3 | | 50 | 100 | | | |
| Note: In Table 13, the MIC for group D2 refers to the concentration of relebactam, and the MIC for groups D3 to D6 refers to the concentration of ceftobiprole. | | | | | | | | | | | | | |

As shown in Tables 12 to 13, the $MIC_{90}$ of taniborbactam alone in group D2 against KPC producing *Klebsiella pneumoniae* and OXA-232 producing *Klebsiella pneumoniae* are both greater than 128 mg/L. Taniborbactam alone shows no antibacterial activity against both KPC producing *Klebsiella pneumoniae* and OXA-232 producing *Klebsiella pneumoniae.*

For KPC producing *Klebsiella pneumoniae,* the $MIC_{50}$ and $MIC_{90}$ of groups D3 to D6 are in the range of 8 to 32 mg/L, and the combination of ceftobiprole and taniborbactam shows certain antibacterial activity against KPC producing *Klebsiella pneumoniae.*

For OXA-232 producing *Klebsiella pneumoniae,* the $MIC_{90}$ of groups D3, D4, and D6 is in the range of 32 to 64 mg/L, and the $MIC_{30}$ and $MIC_{90}$ of group D5 are 64 mg/L and 128 mg/L, respectively. The combination of ceftobiprole and taniborbactam shows weak antibacterial activity against OXA-232 producing *Klebsiella pneumoniae.*

**Comparative Example B4**

An investigation of the combined-use ratio of ceftobiprole and AAI 101 was conducted. The investigation method is the same as in Example 1, with the difference being that zidebactam in the antibacterial drug solutions of groups A2 to A6 in Example 1 is replaced with AAI 101, and the numbering of groups A2 to A6 is correspondingly modified to groups E2 to E6.

The manufacturer of AAI 101 is MCE company, batch number: 40984. The chemical name of AAI 101 is (2*S*,3*S*,5*R*)-3-methyl-3-[(3-methyltriazol-3-ium-1-yl)methyl]-4,4,7-trioxo-4-amino-6-thia-1-azabicyclo[3.2.0]heptane-2-carboxylate; the structural formula is shown as follows:

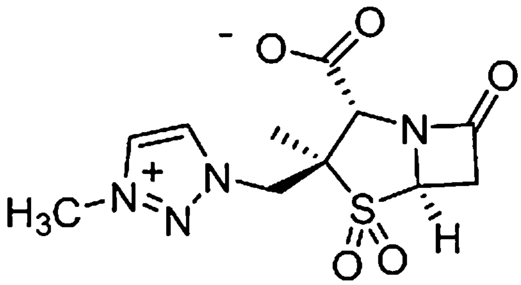

Formula (8)

The results of this comparative example are shown in Tables 14 to 15.

Table 14. *In vitro* antibacterial activity of ceftobiprole/AAI 101 against the tested bacteria

| Bacteria (strains) | Antibacterial drug | MIC (mg/L) | | | |
|---|---|---|---|---|---|
| | | MIC range | $MIC_{50}$ | $MIC_{90}$ | Mode |
| KPC-2 producing *Klebsiella pneumoniae (*12 strains) | Group E2 | >128 | >128 | >128 | >128 |
| | Group E3 | 64 to >128 | 128 | >128 | 128 |
| | Group E4 | 128 to >128 | >128 | >128 | >128 |
| | Group E5 | >128 | >128 | >128 | >128 |
| | Group E6 | >128 | >128 | >128 | >128 |
| OXA-48 producing *Klebsiella pneumoniae (*12 strains) | Group E2 | >128 | >128 | >128 | >128 |
| | Group E3 | 32 to >128 | 128 | >128 | 128 |
| | Group E4 | 64 to >128 | 128 | >128 | 128 |
| | Group E5 | 128 to >128 | >128 | >128 | >128 |
| | Group E6 | 128 to >128 | >128 | >128 | >128 |
| **[0141]** Note: In Table 14, the MIC for group E2 refers to the concentration of relebactam, and the MIC for groups E3 to E6 refers to the concentration of ceftobiprole. | | | | | |

Table 15. MIC distribution and cumulative inhibition rate of ceftobiprole/AAI 101 against the tested bacteria

| Antibacterial drug | | KPC producing MIC (mg/L) | | | OXA producing MIC (mg/L) | | | |
|---|---|---|---|---|---|---|---|---|
| | | 64 | 128 | >128 | 32 | 64 | 128 | >128 |
| Group E2 | Number of strains | | | 12 | | | | 12 |
| | Cumulative number of strains | | | 12 | | | | 12 |
| | Cumulative inhibition rate (%) | | | 100 | | | | 100 |
| Group E3 | Number of strains | 1 | 7 | 4 | 1 | 3 | 5 | 3 |
| | Cumulative number of strains | 1 | 8 | 12 | 1 | 4 | 9 | 12 |
| | Cumulative inhibition rate (%) | 8.3 | 66.7 | 100 | 8.3 | 33.3 | 75 | 100 |
| Group E4 | Number of strains | | 4 | 8 | | 2 | 8 | 2 |
| | Cumulative number of strains | | 4 | 12 | | 2 | 10 | 12 |
| | Cumulative inhibition rate (%) | | 33.3 | 100 | | 16.7 | 83.3 | 100 |
| Group E5 | Number of strains | | | 12 | | | 1 | 11 |
| | Cumulative number of strains | | | 12 | | | 1 | 12 |
| | Cumulative inhibition rate (%) | | | 100 | | | 8.3 | 100 |
| Group E6 | Number of strains | | | 12 | | | 3 | 9 |
| | Cumulative number of strains | | | 12 | | | 3 | 12 |
| | Cumulative inhibition rate (%) | | | 100 | | | 25 | 100 |

Note: In Table 15, the MIC for group E2 refers to the concentration of relebactam, and the MIC for groups E3 to E6 refers to the concentration of ceftobiprole.

As shown in Tables 14 to 15, the $MIC_{50}$ and $MIC_{90}$ of AAI 101 alone in group E2 against KPC producing *Klebsiella pneumoniae* and OXA-232 producing *Klebsiella pneumoniae* are both greater than or equal to 128 mg/L. AAI 101 alone shows no antibacterial activity against both KPC producing *Klebsiella pneumoniae* and OXA-232 producing *Klebsiella pneumoniae.*

For OXA-232 producing *Klebsiella pneumoniae,* the $MIC_{50}$ and $MIC_{90}$ of group E3, group E4, group E5, and group E6 are all greater than or equal to 128 mg/L, and the combination of ceftobiprole and AAI 101 shows no antibacterial activity against OXA-232 producing *Klebsiella pneumoniae.*

Comprehensively comparing Examples 1 to 7, Comparative Example A1, and Comparative Examples B1 to B4, ceftobiprole alone shows no antibacterial activity against KPC producing *Klebsiella pneumoniae* and OXA-232 producing *Klebsiella pneumoniae.* Ceftobiprole in combination with zidebactam shows excellent antibacterial activity against these bacteria, while ceftobiprole in combination with other β-lactamase inhibitors shows no antibacterial activity or weak antibacterial activity against these bacteria. This indicates that the antibacterial composition of the examples of the present disclosure, through the compounding of ceftobiprole and zidebactam, enables the cephalosporin and zidebactam to exert a synergistic effect on each other, thereby effectively enhancing the antibacterial activity of the antibacterial composition.

The examples set forth above merely exemplify several implementation modes of the present disclosure; although the descriptions are relatively specific and detailed, it shall not be construed as a limitation on the patent scope of the present disclosure. It should be noted that, for those of ordinary skill in the art, numerous modifications and improvements can still be made without departing from the conception of the present disclosure, and such modifications and improvements all fall within the protection scope of the present disclosure. Accordingly, the patent protection scope of the present disclosure shall be subject to the appended claims.

## Claims

**1.** An antibacterial composition or an antibacterial kit, wherein the antibacterial composition or the antibacterial kit comprises a cephalosporin and zidebactam;

wherein the cephalosporin comprises at least one of ceftobiprole or a ceftobiprole derivative;
the cephalosporin is calculated as $C_{20}H_{22}N_8O_6S_2$, and the mass ratio of the cephalosporin to zidebactam is 0.1:1 to 10:1.

2. The antibacterial composition or the antibacterial kit according to claim 1, wherein the cephalosporin is calculated as $C_{20}H_{22}N_8O_6S_2$, and the mass ratio of the cephalosporin to zidebactam is 0.1:1 to 8:1, preferably 0.1:1 to 4:1, more preferably 0.1:1 to 2.5:1.

3. The antibacterial composition or the antibacterial kit according to claim 1, wherein the ceftobiprole derivative comprises at least one of a ceftobiprole ester, a ceftobiprole ester derivative, or a ceftobiprole salt;

   preferably, the ceftobiprole salt comprises at least one of an alkali metal salt, hydrochloride, bromate, acetate, or sulfate of ceftobiprole;
   preferably, the alkali metal salt of ceftobiprole comprises at least one of a potassium salt or a sodium salt;
   preferably, the ceftobiprole ester derivative comprises at least one of an alkali metal salt, hydrochloride, bromate, acetate, or sulfate of a ceftobiprole ester;
   preferably, the alkali metal salt of the ceftobiprole ester comprises at least one of a potassium salt or a sodium salt.

4. The antibacterial composition or the antibacterial kit according to any one of claims 1 to 3, wherein the cephalosporin and zidebactam exert a synergistic effect against bacteria;
preferably, the cephalosporin and zidebactam exert a fractional inhibitory concentration index of less than or equal to 0.5 against the bacteria, preferably exert a fractional inhibitory concentration index of less than or equal to 0.2, and more preferably exert a fractional inhibitory concentration index of less than or equal to 0.15.

5. The antibacterial composition or the antibacterial kit according to any one of claims 1 to 4, wherein, when the cephalosporin and zidebactam are used in combination, ceftobiprole has an $MIC_{50}$ of less than or equal to 4 mg/L against the bacteria, preferably an $MIC_{50}$ of less than or equal to 2 mg/L, less than or equal to 0.5 mg/L, less than or equal to 0.125 mg/L, or less than or equal to 0.06 mg/L;
preferably, when the cephalosporin and zidebactam are used in combination, ceftobiprole has an $MIC_{90}$ of less than or equal to 8 mg/L against the bacteria, preferably an $MIC_{90}$ of less than or equal to 4 mg/L, less than or equal to 2 mg/L, less than or equal to 1 mg/L, less than or equal to 0.5 mg/L, or less than or equal to 0.25 mg/L.

6. The antibacterial composition or the antibacterial kit according to any one of claims 1 to 5, wherein the bacteria are Gram-negative bacteria;

   preferably, the bacteria are *Escherichia coli* and/or *Klebsiella pneumoniae;*
   preferably, the bacteria are one or more of NDM producing bacteria, KPC producing bacteria, or OXA producing bacteria.

7. An antibacterial drug, comprising the antibacterial composition or the antibacterial kit as defined in any one of claims 1 to 6.

8. The antibacterial drug according to claim 7, wherein the antibacterial drug comprises a drug used against one or more of NDM producing bacteria, KPC producing bacteria, or OXA producing bacteria; and/or,
the dosage of the antibacterial drug is: the concentration of zidebactam in the application environment is 4 mg/L to 8 mg/L.

9. The antibacterial drug according to claim 8, wherein the NDM producing bacteria comprise *Escherichia coli;* and/or

   the KPC producing bacteria comprise *Klebsiella pneumoniae;* and/or
   the OXA producing bacteria comprise *Klebsiella pneumoniae.*

10. The antibacterial drug according to any one of claims 7 to 9, wherein the antibacterial drug comprises a drug for treating at least one of pulmonary infection, bloodstream infection, or urinary tract infection; and/or
the antibacterial drug comprises an injection.

11. The antibacterial composition or the antibacterial kit as defined in any one of claims 1 to 6 or the antibacterial drug as defined in any one of claims 7 to 10, for use in treating pulmonary infection, bloodstream infection and/or urinary tract infection.

12. Use of the antibacterial composition or the antibacterial kit as defined in any one of claims 1 to 6 or the antibacterial drug as defined in any one of claims 7 to 10 in the manufacture of a medicament for treating pulmonary infection,

bloodstream infection and/or urinary tract infection.

**13.** A method of treating pulmonary infection, bloodstream infection and/or urinary tract infection, comprising administering to a subject in need thereof the antibacterial composition or the antibacterial kit as defined in any one of claims 1 to 6 or the antibacterial drug as defined in any one of claims 7 to 10.

**14.** Use of a therapeutically effective amount of a cephalosporin and a therapeutically effective amount of zidebactam in the manufacture of a medicament, drug combination, or kit for treating pulmonary infection, bloodstream infection and/or urinary tract infection; wherein the therapeutically effective amount of the cephalosporin and zidebactam may be administered simultaneously, separately, or sequentially;

wherein the cephalosporin comprises one or more of ceftobiprole or a ceftobiprole derivative;
the cephalosporin is calculated as $C_{20}H_{22}N_8O_6S_2$, and the mass ratio of the cephalosporin to zidebactam is 0.1:1 to 10:1, preferably 0.1:1 to 8:1, more preferably 0.1:1 to 4:1, more preferably 0.1:1 to 2.5:1.

**INTERNATIONAL SEARCH REPORT**

International application No.
**PCT/CN2024/109115**

**A. CLASSIFICATION OF SUBJECT MATTER**

A61K31/545(2006.01)i; A61K31/454(2006.01)i; A61K45/06(2006.01)i; A61P31/04(2006.01)i

According to International Patent Classification (IPC) or to both national classification and IPC

**B. FIELDS SEARCHED**

Minimum documentation searched (classification system followed by classification symbols)

IPC:A61K; A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, VCN, VEN, CJFD, CNKI, ISI-WEB OF SCIENCE, PUBMED, BING, 头孢比罗, 头孢吡普, 头孢托罗, 齐达巴坦, 肺炎克雷伯菌, 协同, Ceftobiprole, Zevtera, Zidebactam, WCK-5107, WCK 5222, Klebsiella, synergy

**C. DOCUMENTS CONSIDERED TO BE RELEVANT**

| Category* | Citation of document, with indication, where appropriate, of the relevant passages | Relevant to claim No. |
|---|---|---|
| PX | CN 117281812 A (SHENZHEN CHINA RESOURCES GOSUN PHARMACEUTICALS CO., LTD. et al.) 26 December 2023 (2023-12-26)<br>claims 1-10 | 1-14 |
| A | MOYA, B. et al. "In Vitro and In Vivo Activities of β-Lactams in Combination with the Novel β-Lactam Enhancers Zidebactam and WCK 5153 against Multidrug-Resistant Metallo-β-Lactamase-Producing Klebsiella pneumoniae"<br>*Antimicrobial Agents and Chemotherapy,* Vol. 63, No. (5), 25 April 2019 (2019-04-25),<br>pp. 1-9 | 1-14 |
| A | YANG, Y. et al. "In Vitro Activity of Cefepime-1 Zidebactam, Ceftazidime-Avibactam and Other Comparators against Clinical Isolates of Enterobacterales, Pseudomonas aeruginosa and Acinetobacter baumannii: Results from China Antimicrobial Surveillance Network (CHINET) in 2018"<br>*Antimicrobial Agents and Chemotherapy*, Vol. 65, No. (1), 02 November 2020 (2020-11-02),<br>pp. 1-19 | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 November 2024** | **07 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/109115**

**C. DOCUMENTS CONSIDERED TO BE RELEVANT**

| Category* | Citation of document, with indication, where appropriate, of the relevant passages | Relevant to claim No. |
|---|---|---|
| A | SHI, D. et al. "Emergence and Recovery of Ceftazidime-avibactam Resistance in blaKPC-33-Harboring Klebsiella pneumoniae Sequence Type 11 Isolates in China" *Clinical Infectious Diseases,* Vol. vol. 71, 15 November 2020 (2020-11-15), pages S436-S439 | 1-14 |
| A | KHAN, Z. et al. "Activity of Cefepime/Zidebactam (WCK 5222) against Enterobacteriaceae, Pseudomonas aeruginosa and Acinetobacter baumannii Endemic to New York City Medical Centres" *Journal of Antimicrobial Chemotherapy,* Vol. vol. 74, 11 July 2019 (2019-07-11), pages 2938-2942 | 1-14 |
| A | BHAVSAR, S. et al. "Structure Activity Relationship (SAR)" *Medicinal Chemistry Research,* Vol. 32, No. (10), 26 August 2023 (2023-08-26), pages 2245-2255 | 1-14 |
| A | MUSHTAQ, S. et al. "Inoculum Effects of Cefepime/Zidebactam (WCK 5222) and Ertapenem/Zidebactam (WCK 6777) for Enterobacterales in Relation to β-lactamase Type and Enhancer Effect, as Tested by BSAC Agar Dilution" *Journal of Antimicrobial Chemotherapy,* Vol. 77, No. (7), 29 June 2022 (2022-06-29), pages 1916-1922 | 1-14 |
| A | CN 116327764 A (HUASHAN HOSPITAL, FUDAN UNIVERSITY et al.) 27 June 2023 (2023-06-27) claims 1-10 | 1-14 |

Form PCT/ISA/210 (second sheet) (July 2022)

**INTERNATIONAL SEARCH REPORT**

International application No.
**PCT/CN2024/109115**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

The subject matter of claim 13 relates to a method for diagnosing or treating a human body or animal, and therefore said claim does not comply with PCT Rule 39.1(iv). The search report is provided on the basis that the designation of the subject matter of claim 13 is a pharmaceutical use of a corresponding antibacterial compound or antibacterial kit or antibacterial drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/109115**

| Patent document cited in search report | | | Publication date (day/month/year) | Patent family member(s) | Publication date (day/month/year) |
|---|---|---|---|---|---|
| CN | 117281812 | A | 26 December 2023 | None | |
| CN | 116327764 | A | 27 June 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311205221